# EUROPEAN PATENT SPECIFICATION

(11) **EP 3 581 198 B1**
(45) Date of publication and mention of the grant of the patent: **30.03.2022**
(21) Application number: 18884034.2
(22) Date of filing: 07.08.2018
(51) Int. Cl.: A61P 1/16, A61P 3/10, A61K 38/44

(54) **MANGANESE-TYPE HIGH-STABILITY SUPEROXIDE DISMUTASE FOR USE IN TREATING FATTY LIVER**
HOCHSTABILER MANGAN-SUPEROXID-DISMUTASE ZUR VERWENDUNG IN DER BEHANDLUNG VON NICHTALKOHOLISCHER STRATOHEPATITIS
UTILISATION DE SUPEROXYDE DISMUTASE DU TYPE AU MANGANÈSE DE STABILITÉ ÉLEVÉE POUR L'UTILISATION DANS LE TRAITEMENT DE LA STÉATOHÉPATITE NON-ALCOOLIQUE

(30) Priority: 29.11.2017 CN 201711228830
(43) Date of publication of application: 18.12.2019
(73) Proprietor: Hangzhou Redox Pharmatech Co., Ltd, Jiande, Zhejiang 311606 (CN)
(72) Inventor: MENG, Fanguo, Zhejiang 314000 (CN)
(74) Representative: Engelhard, Markus
(86) International application number: PCT/CN2018/099148
(87) International publication number: WO 2019/105060

(56) References cited:
- WO-A1-03/035095
- WO-A1-2017/143850
- CN-A- 103 446 165
- CN-A- 103 505 720
- CN-A- 107 823 635
- R CUI ET AL: "Overexpression of superoxide dismutase 3 gene blocks high-fat diet-induced obesity, fatty liver and insulin resistance", GENE THERAPY, vol. 21, no. 9, 17 July 2014 (2014-07-17), pages 840-848, XP055707884, GB ISSN: 0969-7128, DOI: 10.1038/gt.2014.64
- ADACHI ET AL: "The Forkhead Transcription Factor FoxO1 Regulates Proliferation and Transdifferentiation of Hepatic Stellate Cells", GASTROENTEROLOGY, ELSEVIER INC, US, vol. 132, no. 4, 26 April 2007 (2007-04-26), pages 1434-1446, XP022046106, ISSN: 0016-5085, DOI: 10.1053/J.GASTRO.2007.01.033
- THORSTEN G. LEHMANN ET AL: "EFFECTS OF THREE SUPEROXIDE DISMUTASE GENES DELIVERED WITH AN ADENOVIRUS ON GRAFT FUNCTION AFTER TRANSPLANTATION OF FATTY LIVERS IN THE RAT1 :", TRANSPLANTATION, vol. 76, no. 1, 1 July 2003 (2003-07-01), pages 28-37, XP055707910, GB ISSN: 0041-1337, DOI: 10.1097/01.TP.0000065299.29900.17
- MICHAEL D. WHEELER ET AL: "Overexpression of Manganese Superoxide Dismutase Prevents Alcohol-induced Liver Injury in the Rat", JOURNAL OF BIOLOGICAL CHEMISTRY, vol. 276, no. 39, 28 September 2001 (2001-09-28), pages 36664-36672, XP055707920, US ISSN: 0021-9258, DOI: 10.1074/jbc.M105352200
- Y. C. JANG ET AL: "Overexpression of Mn Superoxide Dismutase Does Not Increase Life Span in Mice", JOURNALS OF GERONTOLOGY, SERIES A, BIOLOGICAL SCIENCES ANDMEDICAL SCIENCES, vol. 64A, no. 11, 1 November 2009 (2009-11-01), pages 1114-1125, XP055707975, US ISSN: 1079-5006, DOI: 10.1093/gerona/glp100
- K. L. HOEHN ET AL: "Insulin resistance is a cellular antioxidant defense mechanism", PROCEEDINGS OF THE NATIONAL ACADEMY OF SCIENCES, vol. 106, no. 42, 20 October 2009 (2009-10-20), pages 17787-17792, XP055707984, ISSN: 0027-8424, DOI: 10.1073/pnas.0902380106
- ZHONG Z ET AL: "Viral gene delivery of superoxide dismutase attenuates experimental cholestasis-induced liver fibrosis in the rat", GENE THERAPY, NATURE PUBLISHING GROUP, LONDON, GB, vol. 9, no. 3, 1 February 2002 (2002-02-01), pages 183-191, XP002425646, ISSN: 0969-7128, DOI: 10.1038/SJ.GT.3301638
- PEREZ V I ET AL: "Is the oxidative stress theory of aging dead?", BIOCHIMICA ET BIOPHYSICA ACTA (BBA) - GENERAL SUBJECTS, ELSEVIER, AMSTERDAM, NL, vol. 1790, no. 10, 1 October 2009 (2009-10-01), pages 1005-1014, XP026586581, ISSN: 0304-4165, DOI: 10.1016/J.BBAGEN.2009.06.003 [retrieved on 2009-06-11]
- JIAN WU ET AL: "Liposome-mediated extracellular superoxide dismutase gene delivery protects against acute liver injury in mice", HEPATOLOGY, vol. 40, no. 1, 1 July 2004 (2004-07-01), pages 195-204, XP055708051, US ISSN: 0270-9139, DOI: 10.1002/hep.20288
- SAMANTHA STEYL: "Overexpression of manganese superoxide dismutase in mouse liver leads to defects in oxidative phosphorylation", EXPERIMENTAL BIOLOGY MEETING, vol. 31, 1 April 2017 (2017-04-01), pages 1-2, XP055708057,
- LAROSCHE ET AL: "[721] OVEREXPRESSION OF MANGANESE SUPEROXIDE DISMUTASE TRIGGERS MITOCHONDRIA!. DAMAGE AFTER CHRONIC ALCOHOL INTOXICATION IN MOUSE LIVER", JOURNAL OF HEPATOLOGY, ELSEVIER, AMSTERDAM, NL, vol. 46, 1 April 2007 (2007-04-01), page S272, XP022088061, ISSN: 0168-8278, DOI: 10.1016/S0168-8278(07)62319-8

## Description

### TECHNICAL FIELD

The present invention belongs to the fields of biomedicine, functional food, etc., and particularly relates to use of a superoxide dismutase.

### BACKGROUND ART

Fatty liver is a widespread chronic disease, which can be divided into alcoholic fatty liver disease (ALD) and non-alcoholic fatty liver disease (NAFLD). Fatty liver is a main cause of liver disease, such as cirrhosis and liver cancer. Non-alcoholic fatty liver disease is characterized by a liver with fat accumulation and metabolic disorders (such as insulin resistance). Hepatic steatosis and metabolic changes caused by long-term high-fat diet or knockout of the obese gene encoding leptin eventually lead to the occurrence of non-alcoholic fatty liver disease, such as liver fibrosis, cirrhosis and liver cancer. Alcoholic fatty liver disease is caused by long-term abuse of alcohol, and can also lead to from a simple steatosis to an end-stage liver disease. The pathogenesis of non-alcoholic fatty liver disease and alcoholic fatty liver disease is very complex, but there are increasing evidences showing that the intestinal barrier injury leads to the translocation of pathogen-associated molecular patterns (pamps) into the extraintestinal tissue, accompanying with the changes of intestinal flora and the occurrence of NAFLD and ALD. Although intestinal barrier injury is known to be an important driver of fatty liver development, there are few effective ways to treat fatty liver symptoms by repairing intestinal barrier injury.

Free radicals have been thought to be involved in the destruction of intestinal barrier for a long time, but few people paid attention to free radicals in the intestinal contents. In vitro studies have shown that intracellular and extracellular superoxide radicals can disrupt the monolayer structure of Caco-2 cells. Currently, extracellular superoxide is thought to be mainly derived from inflammatory cells including macrophages and monocytes. On the other hand, the analysis of metagenomics show that the oxidative stress of the intestinal flora of patients with metabolic diseases was significantly increased. It is worth noting that, in the rat model, the *Enterococcus faecalis* strain produces extracellular superoxide and produces hydroxyl radicals. Despite the lack of direct evidence showing that the intestinal flora produces superoxide, intestinal microbes may be another source of extracellular superoxide and affect the local environment of intestinal tract. Recent studies have shown that high-fat diet feeding is closely related to the increase of reactive oxygen species (ROS) of intestinal microbes, but the details of the influence of active oxygen of the intestinal flora on the development of fatty liver remain to be studied.

Steyl, Experimental Biology Meeting, 2017, Volume 31, April 1, 2017, pages 1-2, XP055708057 discloses an overexpression of manganese superoxide dismutase in mouse liver and shows that this leads to defects in oxidative phosphorylation. The data by Steyl indicate that antioxidant therapy may have unexpected complications that should be considered before administration of these drugs.

### SUMMARY OF THE INVENTION

The inventors of the present application have investigated the potential effects of intestinal bacterial superoxide on fatty liver and proposed a new approach to treat fatty liver.

Specifically, the present invention provides a manganese superoxide dismutase with high stability (MS-SOD) for use in treating fatty liver, wherein the amino acid sequence of the manganese superoxide dismutase with high stability is set forth in SEQ ID NO: 4.

In a specific embodiment, the fatty liver is alcoholic fatty liver or non-alcoholic fatty liver.

In a specific embodiment, the fatty liver is fatty liver accompanied with metabolic syndrome.

Preferably, the metabolic syndrome is hyperglycemia or insulin resistance.

Preferably, the medicament is administered by oral, injection or intragastric route. Further preferably, the injection is intravenous injection or intraperitoneal injection. Preferably, the dosage form of the medicament is a tablet, a capsule, a powder injection, an injection or an aerosol.

Preferably, the medicament further comprises one or more pharmaceutically acceptable excipients, such as diluents, binders, wetting agents, disintegrating agents, solvents and/or buffers, and the like. These excipients are prepared as a medicament in a manner well known in the art with MS-SOD, and the amounts thereof are also known to those skilled in the art.

The manganese superoxide dismutase with high stability provided by the invention can effectively reduce the superoxide level, and has a good therapeutic effect on various types of fatty liver, such as alcoholic fatty liver, non-alcoholic fatty liver, fatty liver accompanied with metabolic syndrome.

### DESCRIPTION OF THE DRAWINGS

Figure 1A shows gene abundance analysis of enzymes associated with oxidative stress;
Figure 1B shows relative superoxide levels in mouse intestinal feces (n = 8-14);
Figure 2A shows total SOD activity in plasma (n = 8);
Figure 2B shows total SOD activity in intestinal wall (n = 8);
Figure 2C shows total SOD activity in cecal contents (n = 7-8);
Figure 3A shows weight comparison between the high-fat diet group and the MS-SOD treatment group;
Figure 3B shows relative weights of adipose tissues;
Figure 3C is a graph of HE and oil red O staining for liver (n = 6-9);
Figure 3D is fatty liver scores (n = 6-9);
Figure 3E shows total triglyceride contents in liver (n = 7-11);
Figure 3F shows the mRNA relative expression levels of hepatocyte cytokines and chemokines (n = 10-15);
Figure 3G is the glucose tolerance test (GTT) results (n = 10-20);
Figure 3H is the insulin tolerance test (ITT) results (n = 10-20);
Figure 3I shows fasting blood glucose levels (n=10-20);
Figure 3J shows fasting insulin levels (n=10-20);
Figure 3K shows homeostasis model assessment of insulin resistance HOMA-IR index levels (n=10-20);
Figure 4A shows ethanol concentrations in plasma;
Figure 4B shows mRNA levels of alcohol dehydrogenase (Adh1) and cytochrome P4502E1 (Cyp2e1) in liver;
Figure 4C shows ratios of liver to body weight (n =8-10);
Figure 4D shows alanine aminotransferase levels in plasma (n=8-10);
Figure 4E shows the results of HE and oil red O staining for liver (n=6-10);
Figure 4F shows the fatty liver scores based on oil red O staining (n=6-10);
Figure 4G shows the total triglyceride contents in liver (n=7-10);
Figure 4H shows the relative expression levels of Ccl2 mRNA in liver (n=8-10);
Figure 4I shows the relative amounts of superoxide in intestinal feces (n=6-8);
Figure 4J shows PCoA analysis of cecal bacteria (n=6-8);
Figure 4K shows fecal albumin levels (n= 8-11);
Figure 4L shows endotoxin levels in plasma (n=8-11);
Figure 5A shows changes in body weight;
Figure 5B shows the relative weights of adipose tissues;
Figure 5C shows the results of HE and oil red O staining for liver;
Figure 5D is fatty liver scores;
Figure 5E shows the total triglyceride contents in liver;
Figure 5F shows Ccl4 mRNA levels in liver;
Figure 5G shows the glucose tolerance test (GTT) results;
Figure 5H shows the insulin tolerance test (ITT) results;
Figure 5I is a diagram of the variation of the classification of the intestinal flora;
Figure 5J shows fecal albumin levels;
Figure 5K shows endotoxin lipopolysaccharide levels in plasma.

### DETAILED DESCRIPTION OF THE INVENTION

In the following examples, unless otherwise specified, the detection and monitoring of various indicators were carried out by methods known in the art.

Among them, the kits for the determination of plasma insulin, glucose, total cholesterol and blood ethanol were all purchased from Nanjing JianCheng Bioengineering Institute. The LPS assay kit was purchased from Bioswamp.

The mice used in the examples were all purchased from the Guangdong Experimental Animal Center.

Glucose Tolerance Test (GTT) and Insulin Tolerance Test (ITT): Refer to "Principles and Clinical Applications of Glucose Tolerance Test", Shanghai Med J. 2009, Vol. 32, No. 5, pp. 440-443.

### Example 1: Preparation of Manganese Superoxide Dismutase with High Stability (MS-SOD)

Using the genome of the *Thermus thermophilus* HB27 (purchased from the ATCC cell bank of the United States, accession number *ATCC BAA-163)* as a template, the following primers were used to carry out the amplification: forward primer: 5'-aagaattcatgccgtacccgttcaagct-3' (SEQ ID NO:1) and reverse primer: 5'-ctgtcgactcaggctttgttgaagaac-3' (SEQ ID NO: 2), to obtain the target gene; the amplified product was recovered using a recovery kit (purchased from Sangon Biotech (Shanghai) Co., Ltd.), double-digested with enzymes EcoRI and *Sal* I and ligated into the plasmid vector pET28a(+) (purchased from Sangon Biotech (Shanghai) Co., Ltd.) which was double-digested with the same enzymes. The recombinant plasmid was transformed into competent *E. coli* BL21 (DE3) (purchased from Sangon Biotech (Shanghai) Co., Ltd.). The strain with the correct MS-SOD nucleotide sequence was screened by sequencing, and the strain was cultured to obtain MS-SOD protein. Wherein, the nucleotide sequence of the gene encoding MS-SOD is as follows:

The amino acid sequence of MS-SOD is as follows:

### Example 2: Effect of MS-SOD on intestinal flora in mice induced by high-fat diet

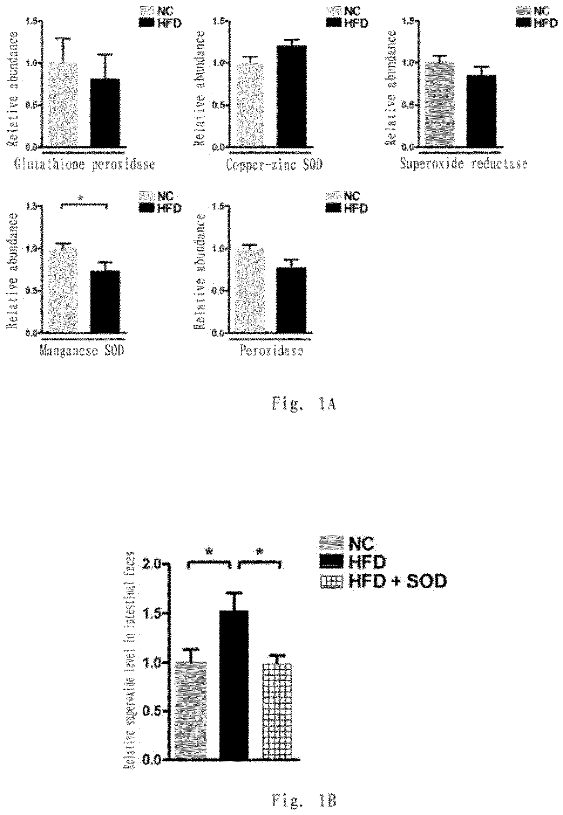

1. Establishment of animal models:
   Male C57BL/6J mice with 6-8 weeks old (purchased from Guangdong Experimental Animal Center) were divided into 3 groups:
   (1) Normal control group (NC): fed with low-fat diet (fat-derived calorie 10%) for 18 weeks;
   (2) High-fat diet group (HFD): fed with high-fat diet (fat-derived calorie 60%) for 18 weeks;
   (3) High-fat diet +MS-SOD group (HFD+MS-SOD): the mice were fed with high-fat diet for 18 weeks, and administered with 3 U MS-SOD /g body weight by intragastric administration from the 8th week. The dose of the intragastric administration was 0.1ml per mouse, once a day, until the 18th week. The MS-SOD was obtained from the preparation as shown in Example 1.
   The components of low-fat diet: corn flour, soybean meal, wheat flour, fish, vegetable oil, etc.
   The components of high-fat diet: lard was added to the low-fat diet.
2. Experimental contents:
   (1) Detection of enzymes associated with oxidative stress
      A biotechnology company was entrusted to obtain the gene abundances of manganese superoxide dismutase (Mn-SOD), copper-zinc superoxide dismutase (CuZn-SOD), glutathione peroxidase, catalase and superoxide reductase, by performing metagenomics sequencing on the intestinal flora of NC group and HFD group mice. Specifically:
      a. The feces of NC group and HFD group mice were taken separately, 1g per mouse and then were suspended in 5 ml of 50 mM phosphate buffer (containing 0.5% Tween-20). The resulting samples were repeatedly frozen and thawed at -80°Cand 60°C for 3 times, to destroy microbial cells.
      b. Total RNA of the samples was extracted using Trizol method kit of Invitrogen, and samples were detected by real-time quantitative fluorescent PCR using the ABI 7500 rtPCR system.
      c. Determination of the relative expression of the enzyme genes: using 18sRNA as the internal reference gene, the target gene and the internal reference gene were simultaneously amplified by quantitative PCR, and then the relative expression of the target gene was analyzed by Ct comparison method. Primer sequences were obtained from the NIH qPrimerdepot database.
   (2) Detection of superoxide levels:
      The test was carried out using the superoxide detection kit of Beyotime Biotechnology.
      The principle of the detection is: superoxide can be detected by using the superoxide to reduce water-soluble tetrazolium-1 (WST-1) to produce soluble colored substances.
         a. Adding 200 µ1 of superoxide buffer, 10 µ1 of WST-1 solution, and 2 µ1 of Catalase solution to the detection wells of the ELISA plate;
         b. Adding the processed sample to be tested to the detection wells;
         c. Incubating for 3 minutes at 37°C; absorbance was measured at 450 nm.
3. Experimental results
   1) High-fat diet feeding increased the superoxide level of intestinal microbes in mice. Studies on genes related to reactive oxygen metabolism in mice, including manganese superoxide dismutase, copper-zinc superoxide dismutase, glutathione peroxidase, catalase and superoxide reductase genes were conducted and it was found that except for copper-zinc SOD, all of the gene abundances of other enzymes decreased (Fig. 1A). However, the level of superoxide in the intestinal flora of mice was significantly increased (Fig. 1B). These data clearly indicated that high-fat diet can increase the levels of superoxide in intestinal microbes and reduce the abundances of genes involved in reactive oxygen metabolism.
   2) MS-SOD had a repairing effect on the imbalance of superoxide level of intestinal flora induced by high-fat diet.

As above, in order to study the effect of MS-SOD, comparative experiments were conducted at the same time. The results showed that compared with the high-fat diet group mice, the superoxide level in the MS-SOD (which was administered intragastrically) group was reduced to normal level (Fig. 1B), indicating that MS-SOD can repair the imbalance of superoxide level of intestinal flora induced by high-fat diet.

### Example 3: Effect of intragastric administration and injection of MS-SOD on SOD

### activity in intestinal tract of mice

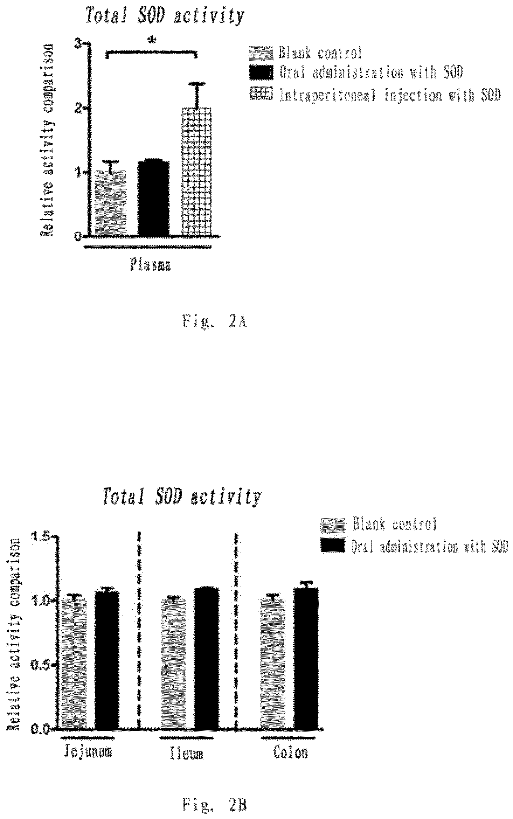

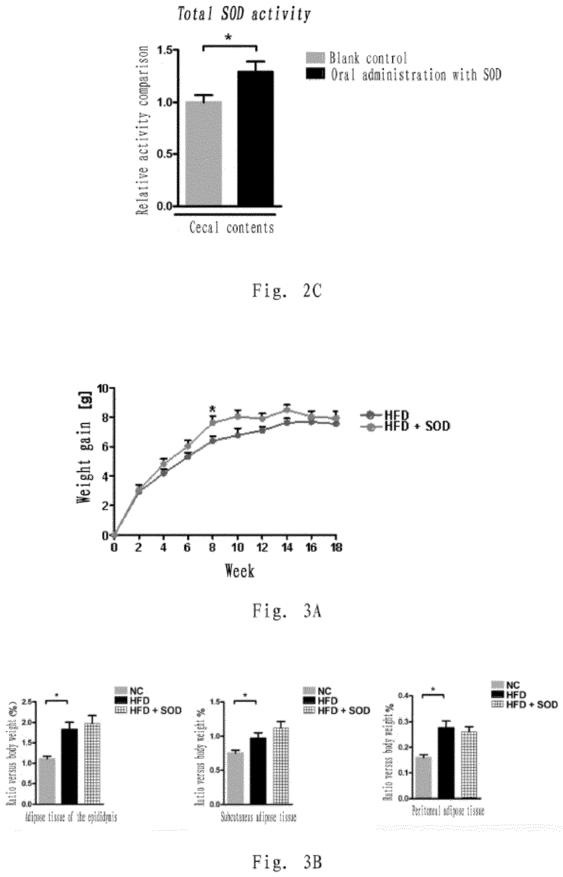

1. Establishment of animal models:
   Male C57BL/6J mice with 6-8 weeks old (purchased from Guangdong Experimental Animal Center) were divided into 3 groups:
   (1) MS-SOD intragastric administration group: the mice were intragastrically (orally) administered with 6 U MS-SOD /g body weight of per day, and the dose of the intragastric administration was 0.1 ml per mouse, once a day for 7 consecutive days;
   (2) Phosphate buffer control group: the mice were administered intragastrically (orally) with a dose of 0.1 ml of phosphate buffer per day, once a day for 7 consecutive days;
   (3) MS-SOD intraperitoneal injection group: the mice were intraperitoneally injected with 6 U MS-SOD/g body weight per day, and the dose of the injection was 0.1 ml per mouse, once a day for 7 consecutive days.
2. Experimental contents:
   After 7 days, the plasma, jejunum, ileum, colon and cecal contents of each group of experimental mice were taken, and pyrogallol method was used (refer to GB/T5009.171-2003 "Determination of Superoxide Dismutase (SOD) Activity in Health Foods ") to detect the activity of SOD.
3. Experimental results:
   As shown in Fig. 2A, injection of MS-SOD caused an increase of total SOD activity in plasma; in contrast, administering intragastrically (orally) with MS-SOD did not significantly change total SOD in plasma in mice, indicating that oral MS-SOD did not affect extraintestinal organs. As shown in Fig. 2B, the total SOD activity in the jejunum, ileum and colon did not change significantly; however, as shown in Fig. 2C, the SOD activity in the cecal contents was significantly enhanced, indicating that MS-SOD can directly change the oxidation reduction potential in the intestinal bacterial environment.

### Example 4: Effect of MS-SOD on lipid accumulation of liver and metabolic syndrome in mice with high-fat diet

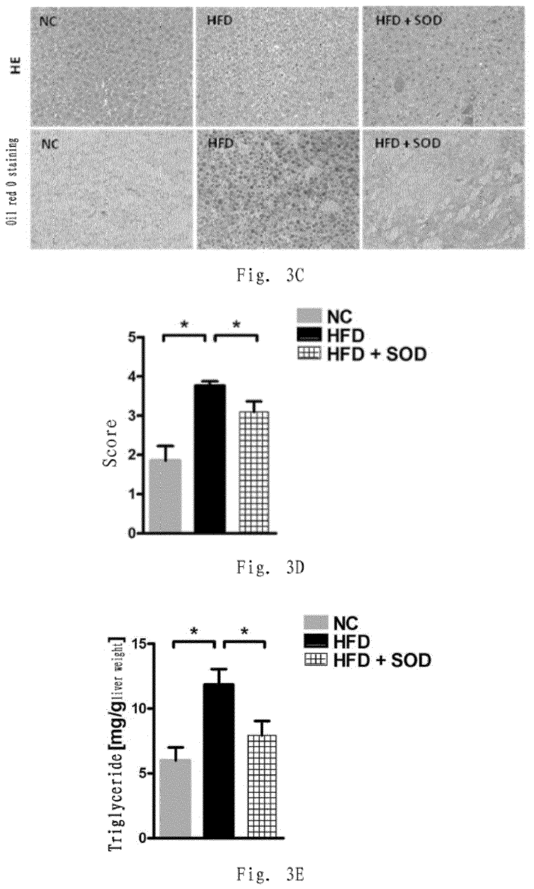

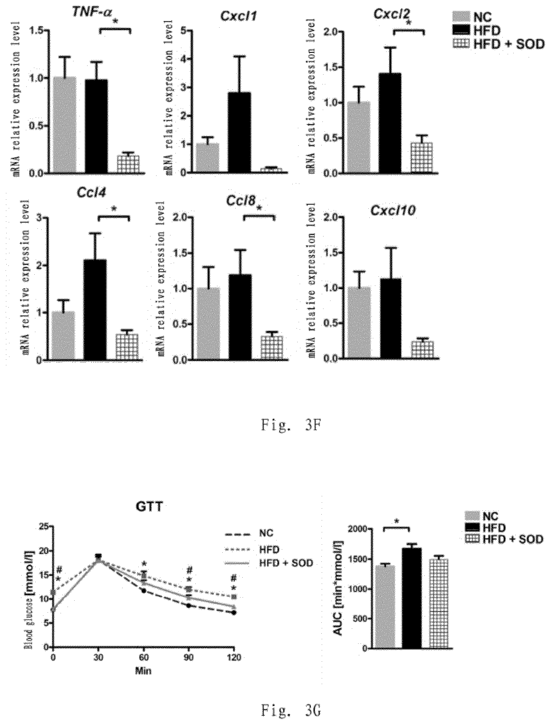

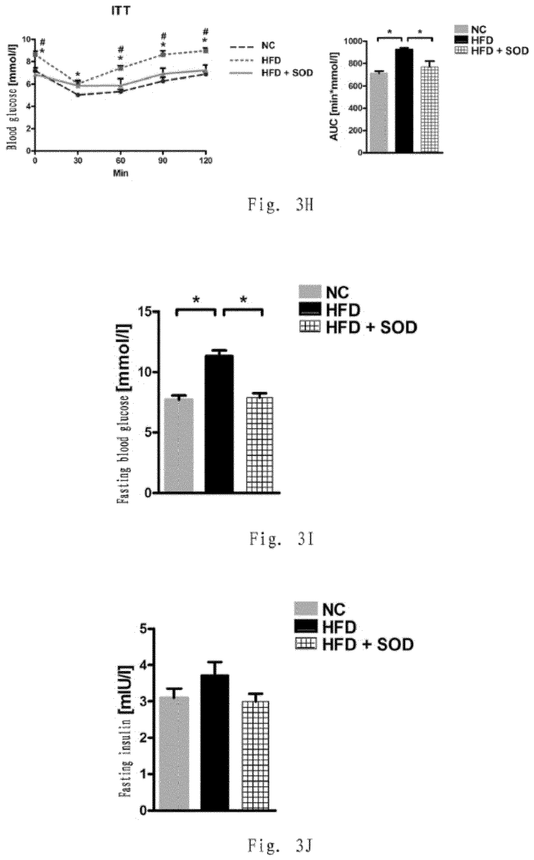

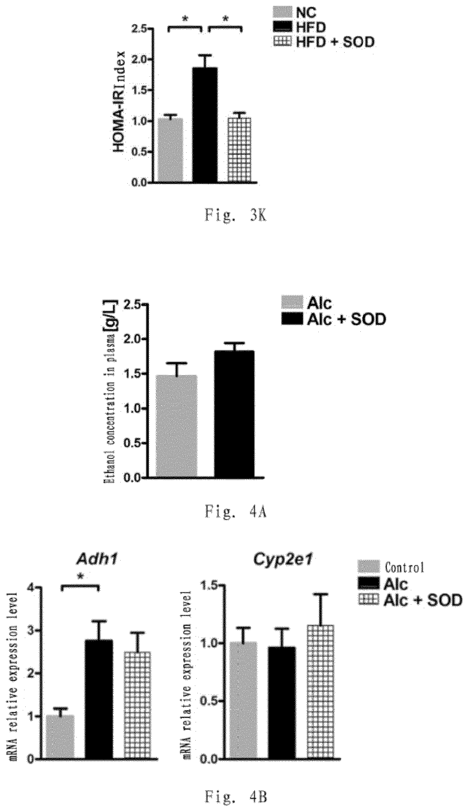

1. Establishment of animal models:
   The establishment of animal models was the same as that in Example 2.
2. Experimental results
   (1) The body weights and the adipose tissue contents of the mice were examined. As shown in Figs. 3A and 3B, administering intragastrically with MS-SOD did not alter the body weights and adipose tissue contents of the mice. However, it can be seen from the liver section that long-term high-fat diet led to hepatic steatosis. After administering intragastrically with MS-SOD, the fatty liver symptoms were significantly relieved (Figs. 3C-D), and the triglyceride content of liver was significantly reduced (Fig. 3E).
   (2) The mRNA of liver tissue was extracted and reverse transcribed into cDNA. The expression of key cytokines in liver was studied by real-time fluorescent quantitative PCR. The results showed that MS-SOD significantly reduced the mRNA levels of tumor necrosis factor tnf-α, chemokines ccl4, ccl8, cxc11, cxcl2 and cxc110 during high-fat diet feeding (Fig. 3F).
   (3) High-fat diet feeding also led to metabolic disorders, such as glucose tolerance and insulin resistance. Glucose tolerance test (GTT) showed that, compared with the mice fed with high-fat diet, the blood glucose levels were significantly reduced in mice administered intragastrically with MS-SOD (Fig. 3G). Consistent with this, insulin tolerance test results showed that, compared with the mice fed simply with high-fat diet, the mice fed with high-fat diet and administered intragastrically with MS-SOD were more sensitive to insulin (Fig. 3H). Compared with the normal control group, high-fat diet feeding significantly increased fasting blood glucose level and HOMA-IR index, which was a marker of insulin resistance. MS-SOD can almost restore these parameters to normal (Figs. 3I-K). MS-SOD can improve the impaired insulin sensitivity induced by high-fat diet. It can be seen that MS-SOD can significantly reduce hepatic steatosis and metabolic disorders caused by high-fat diet.

### Example 5: MS-SOD alleviated the symptoms of alcoholic fatty liver

### I. Experimental protocol

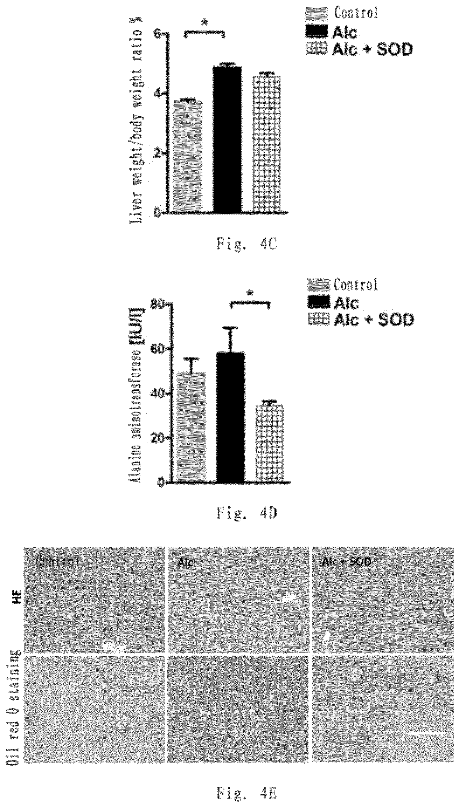

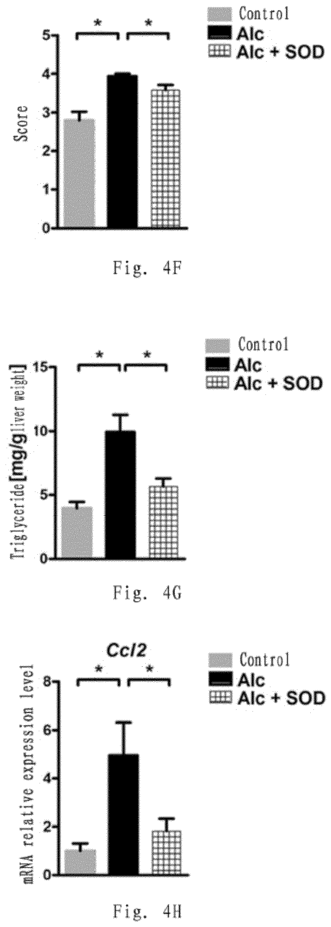

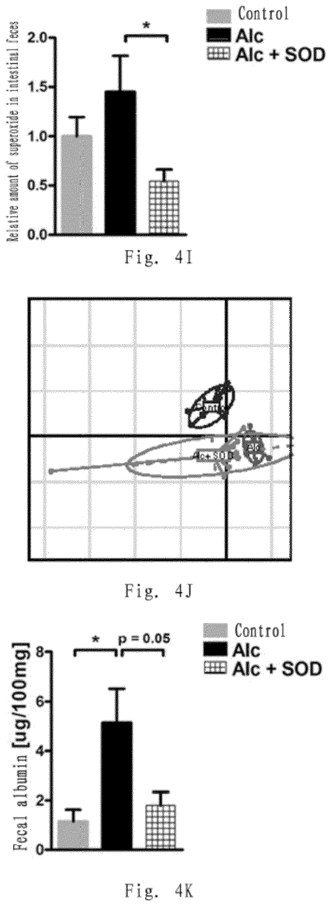

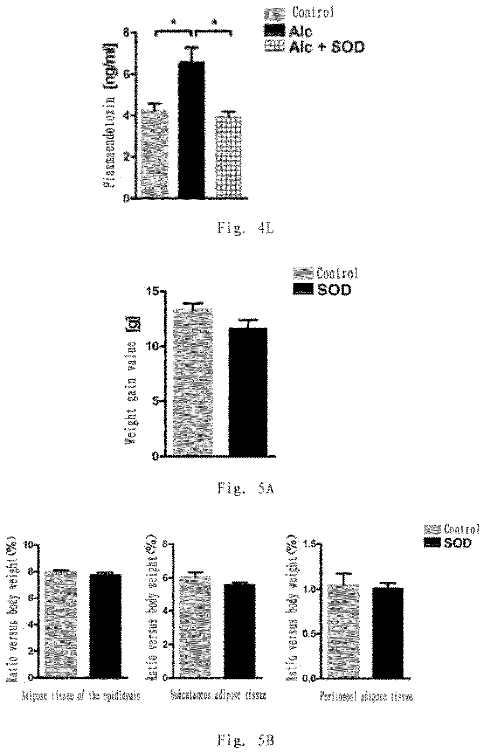

1. Animal models:
   60 male C57 mice with 8 weeks old (purchased from the Guangdong Experimental Animal Center) were housed in a SPF environment and divided into 3 groups:
   Blank control group (control): fed with the Lieber-DeCarli alcohol-free liquid diet for 9 weeks;
   Model control group: (alcohol, Alc): fed with the Lieber-DeCarli alcoholic liquid diet for 9 weeks;
   MS-SOD treatment group (Alc+SOD): fed with the Lieber-DeCarli alcoholic liquid diet for 9 weeks, while administered intragastrically with MS-SOD (3 U/g body weight). Lieber-DeCarli alcoholic liquid feed (TP4231C, calories including fat 40%, protein 18%, carbohydrate 14%, alcohol 28%, purchased from Trophic Animal Feed High-Tech Co., Ltd, China).
2. Experimental protocol:
   The MS-SOD treatment group was administered intragastrically with MS-SOD solution (3 U/g body weight), the amount of intragastric administration was 0.1 ml per mouse, once a day, and the period of intragastric administration was 9 weeks. The blank control group and the model control group were administrated intragastrically with sterile water, and the amount, frequency and period of intragastric administration were the same as those in the treatment group. During the experiment period, fresh feces were collected at 9 am Friday of every two weeks and stored at -80°C. At the 4th, 6th and 8th week of the intragastric intervention, the ALT/AST levels in plasma in mice were monitored by blood collection from eye orbit. On the last day of the experiment, the mice were dissected and tissues were taken and frozen at -80 °C.
3. Experimental results:
   The mice were induced for eight weeks by the liber DeCarli diet. Ethanol metabolism was not affected by MS-SOD, such as ethanol level in plasma and alcohol dehydrogenase, cytochrome p4502E1 expression in liver (Figs. 4A, 4B). The liver weight/body weight ratio was significantly higher in the alcohol group, and compared with the alcohol group, the liver weight/body weight ratio in the alcohol+SOD group was slightly lower (Fig. 4C). At the same time, alanine aminotransferase level in plasma was significantly reduced in the alcohol+SOD group (Fig. 4D). Importantly, hepatic lipid accumulation and liver ccl2 expression were significantly reduced in the MS-SOD group (Figs. 4E-H). Chronic alcohol abuse showed a clear trend of a higher level of superoxide in the feces, but MS-SOD can significantly reduce superoxide level during alcohol feeding (Fig. 4I). Finally, administering intragastrically with MS-SOD significantly reduced the elevated intestinal permeability and translocation of microbial product caused by chronic alcohol (Figs. 4J-L). In summary, MS-SOD can improve the symptoms, such as intestinal flora imbalance, intestinal leakage and hepatic steatosis caused by alcohol feeding.

### Example 6: MS-SOD alleviated symptoms of fatty liver and metabolic syndrome in leptin gene deficient mice (ob/ob mice)

### I. Experimental protocol

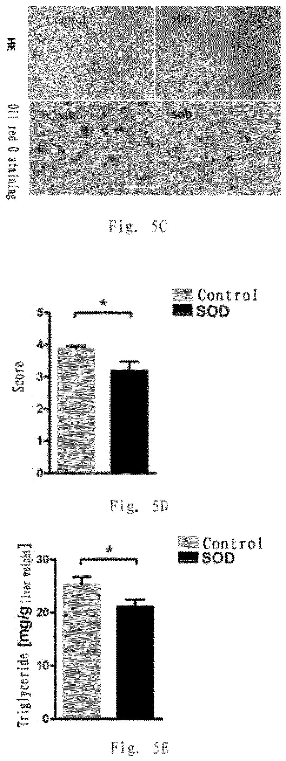

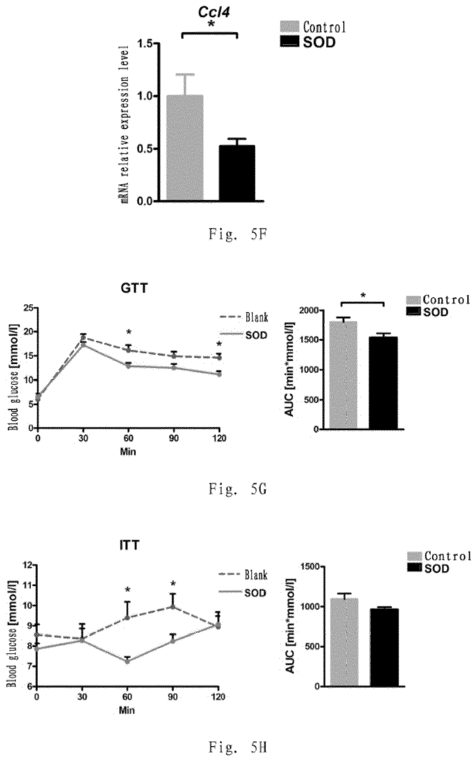

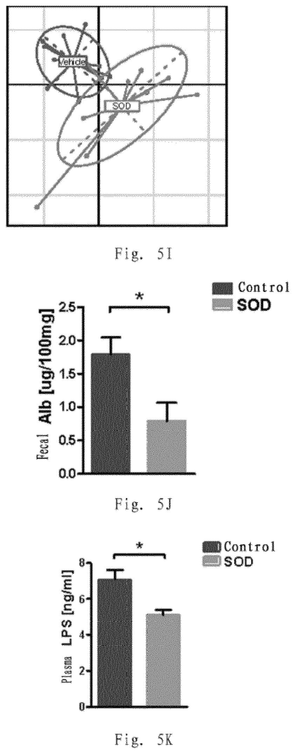

1. Animal models:
   Leptin gene deficient mice (ob/ob mice): 6-8 weeks old male mice, purchased from the Guangdong Experimental Animal Center.
2. Experimental protocol
   (1) ob/ob mice were fed with MS-SOD (3 U/g body weight) from the 8th week until the 12th week.
   (2) Glucose tolerance test (GTT): mice were injected intraperitoneally with glucose (1 g/kg body weight) after 16 hours of fasting. Blood glucose concentrations were measured at 0, 30, 60, 90, and 120 minutes, respectively.
   (3) Insulin tolerance test (ITT): mice were injected intraperitoneally with insulin (0.35 U/kg body weight) after 6 hours of fasting. Blood glucose concentrations were measured at 0, 30, 60, 90, and 120 minutes, respectively.
3. Experimental results
   MS-SOD treatment alleviated the fatty liver and metabolic syndrome of leptin gene deficient mice (ob/ob mice). MS-SOD did not significantly change the body weights of ob/ob mice (Figs. 5A, B), but the adipose tissue index was significantly improved (Figs. 5C, D), and the triglyceride content of liver was also significantly reduced (Fig. 5E). The mRNA level of liver Ccl4 was also significantly reduced (Fig. 5F). GTT and ITT experiments demonstrated that administering intragastrically with MS-SOD resulted in a better control of blood glucose in mice, a significant reduction in blood glucose, and a greater sensitivity to insulin (Figs. 5G, H). Importantly, similar to the high-fat diet feeding model, MS-SOD treatment significantly changed the composition of the intestinal flora (Fig. 5I). By monitoring the levels of fecal albumin and endotoxin lipopolysaccharide in plasma, it was found that MS-SOD significantly improved intestinal leakage and translocation of bacterial products (Figs. 5J, K). In conclusion, MS-SOD can relieve the symptoms of fatty liver and metabolic disorders in the OB/OB mouse model.

## Claims

1. A manganese superoxide dismutase with high stability for use in treating fatty liver, wherein the amino acid sequence of the manganese superoxide dismutase with high stability is set forth in SEQ ID NO: 4.

2. The manganese superoxide dismutase with high stability for use according to claim 1, wherein the fatty liver is alcoholic fatty liver or non-alcoholic fatty liver.

3. The manganese superoxide dismutase with high stability for use according to claim 1 or 2, wherein the fatty liver is a fatty liver accompanied with metabolic syndrome.

4. The manganese superoxide dismutase with high stability for use according to claim 3, wherein the metabolic syndrome is hyperglycemia or insulin resistance.

5. The manganese superoxide dismutase with high stability for use according to any one of claims 1 to 4, wherein the medicament is administered by oral, injection or intragastric route.

6. The manganese superoxide dismutase with high stability for use according to claim 5, wherein the injection is intravenous injection or intraperitoneal injection.

7. The manganese superoxide dismutase with high stability for use according to any one of claims 1 to 4, wherein the dosage form of the medicament is a tablet, a capsule, a powder injection, an injection or an aerosol.

8. The manganese superoxide dismutase with high stability for use according to any one of claims 1 to 4, wherein the medicament further comprises one or more pharmaceutically acceptable excipients.

9. The manganese superoxide dismutase with high stability for use according to claim 8, wherein the excipient is a diluent, a binder, a wetting agent, a disintegrating agent, a solvent and/or a buffer.

## Patentansprüche

1. Mangan-Superoxid-Dismutase mit hoher Stabilität zur Verwendung bei einem Behandeln einer Fettleber, wobei die Aminosäuresequenz der Mangan-Superoxid-Dismutase mit hoher Stabilität in SEQ ID NO: 4 dargelegt ist.

2. Mangan-Superoxid-Dismutase mit hoher Stabilität zur Verwendung nach Anspruch 1, wobei die Fettleber eine alkoholische Fettleber oder eine nichtalkoholische Fettleber ist.

3. Mangan-Superoxid-Dismutase mit hoher Stabilität zur Verwendung nach Anspruch 1 oder 2, wobei die Fettleber eine mit einem metabolischen Syndrom einhergehende Fettleber ist.

4. Mangan-Superoxid-Dismutase mit hoher Stabilität zur Verwendung nach Anspruch 3, wobei das metabolische Syndrom eine Hyperglykämie oder Insulinresistenz ist.

5. Mangan-Superoxid-Dismutase mit hoher Stabilität zur Verwendung nach einem der Ansprüche 1 bis 4, wobei das Arzneimittel oral, durch Injektion oder auf einem intragastrischen Weg verabreicht wird.

6. Mangan-Superoxid-Dismutase mit hoher Stabilität zur Verwendung nach Anspruch 5, wobei die Injektion eine intravenöse Injektion oder eine intraperitoneale Injektion ist.

7. Mangan-Superoxid-Dismutase mit hoher Stabilität zur Verwendung nach einem der Ansprüche 1 bis 4, wobei die Darreichungsform des Arzneimittels eine Tablette, eine Kapsel, eine Pulverinjektion, eine Injektion oder ein Aerosol ist.

8. Mangan-Superoxid-Dismutase mit hoher Stabilität zur Verwendung nach einem der Ansprüche 1 bis 4, wobei das Arzneimittel weiter einen oder mehrere pharmazeutisch akzeptable Hilfsstoffe umfasst.

9. Mangan-Superoxid-Dismutase mit hoher Stabilität zur Verwendung nach Anspruch 8, wobei der Hilfsstoff ein Verdünnungsmittel, ein Bindemittel, ein Benetzungsmittel, ein Sprengmittel, ein Lösungsmittel und/oder ein Puffer ist.

## Revendications

1. Superoxyde dismutase à manganèse de stabilité élevée destinée à être utilisée dans le traitement de la stéatohépatite, dans laquelle la séquence d'acides aminés de la superoxyde dismutase à manganèse de stabilité élevée est décrite dans SEQ ID NO: 4.

2. Superoxyde dismutase à manganèse de stabilité élevée destinée à être utilisée selon la revendication 1, dans laquelle la stéatohépatite est la stéatohépatite alcoolique ou la stéatohépatite non alcoolique.

3. Superoxyde dismutase à manganèse de stabilité élevée destinée à être utilisée selon la revendication 1 ou 2, dans laquelle la stéatohépatite est une stéatohépatite accompagnée d'un syndrome métabolique.

4. Superoxyde dismutase à manganèse de stabilité élevée destinée à être utilisée selon la revendication 3, dans laquelle le syndrome métabolique est l'hyperglycémie ou l'insulinorésistance.

5. Superoxyde dismutase à manganèse de stabilité élevée destinée à être utilisée selon l'une quelconque des revendications 1 à 4, dans laquelle le médicament est administré par voie orale, par injection ou par voie intragastrique.

6. Superoxyde dismutase à manganèse de stabilité élevée destinée à être utilisée selon la revendication 5, dans laquelle l'injection est une injection intraveineuse ou une injection intrapéritonéale.

7. Superoxyde dismutase à manganèse de stabilité élevée destinée à être utilisée selon l'une quelconque des revendications 1 à 4, dans laquelle la forme posologique du médicament est un comprimé, une capsule, une poudre pour solution injectable, une injection ou un aérosol.

8. Superoxyde dismutase à manganèse de stabilité élevée destinée à être utilisée selon l'une quelconque des revendications 1 à 4, dans laquelle le médicament comprend en outre un ou plusieurs excipients pharmaceutiquement acceptables.

9. Superoxyde dismutase à manganèse de stabilité élevée destinée à être utilisée selon la revendication 8, dans laquelle l'excipient est un diluant, un liant, un agent mouillant, un délitant, un solvant et/ou un tampon.


### SEQUENCE LISTING

<110> HANGZHOU REDOX PHARMATECH CO., LTD
<120> USE OF MANGANESE SUPEROXIDE DISMUTASE WITH HIGH STABILITY
<130> H33418WOEP
<140> EP18884034.2
   <141> 2018-08-07
<150> CN201711228830
   <151> 2017-11-29
<160> 4
<170> SIPOSequenceListing 1.0
<210> 1
   <211> 28
   <212> DNA
   <213> Artificial Sequence
<220>
   <223> Synthetic Primer
<400> 1
   aagaattcat gccgtacccg ttcaagct 28
<210> 2
   <211> 27
   <212> DNA
   <213> Artificial Sequence
<220>
   <223> Synthetic Primer
<400> 2
   ctgtcgactc aggctttgtt gaagaac 27
<210> 3
   <211> 615
   <212> DNA
   <213> Thermus thermophilus
<400> 3
   atgccgtacc cgttcaagct tcctgaccta ggctacccct acgaggccct cgagccccac        60
   attgacgcca agaccatgga gatccaccac cagaagcacc acggggccta cgtgacgaac       120
   ctcaacgccg ccctggagaa gtacccctac ctccacgggg tggaggtgga ggtcctcctg       180
   aggcacctcg ccgcccttcc ccaggacatc cagaccgccg tgcgcaacaa cgggggcggg       240
   cacctgaacc acagcctctt ctggaggctc ctcacccccg ggggggccaa ggagcccgtg        300
   ggggagctga agaaggccat tgacgagcag ttcgggggct tccaggccct caaggagaag       360
   ctcacccagg cggccatggg ccggttcggc tcgggctggg cctggctcgt gaaggacccc       420
   ttcggcaagc tccacgtcct ctccaccccc aaccaagaca accccgtgat ggagggcttc       480
   acccccatcg tgggcattga cgtctgggag cacgcctact acctcaagta ccagaaccgc       540
   cgggccgatt acctccaggc catctggaac gtcctcaact gggacgtggc cgaggagttc       600
   ttcaataaag cctga                                                          615
<210> 4
   <211> 204
   <212> PRT
   <213> Thermus thermophilus
<400> 4
   Met Pro Tyr Pro Phe Lys Leu Pro Asp Leu Gly Tyr Pro Tyr Glu Ala
   1               5                   10                  15
   Leu Glu Pro His Ile Asp Ala Lys Thr Met Glu Ile His His Gln Lys
               20                  25                  30
   His His Gly Ala Tyr Val Thr Asn Leu Asn Ala Ala Leu Glu Lys Tyr
           35                  40                  45
   Pro Tyr Leu His Gly Val Glu Val Glu Val Leu Leu Arg His Leu Ala
       50                  55                  60
   Ala Leu Pro Gln Asp Ile Gln Thr Ala Val Arg Asn Asn Gly Gly Gly
   65                  70                  75                  80
   His Leu Asn His Ser Leu Phe Trp Arg Leu Leu Thr Pro Gly Gly Ala
                   85                  90                  95
   Lys Glu Pro Val Gly Glu Leu Lys Lys Ala Ile Asp Glu Gln Phe Gly
               100                 105                 110
   Gly Phe Gln Ala Leu Lys Glu Lys Leu Thr Gln Ala Ala Met Gly Arg
           115                 120                 125
   Phe Gly Ser Gly Trp Ala Trp Leu Val Lys Asp Pro Phe Gly Lys Leu
       130                 135                 140
   His Val Leu Ser Thr Pro Asn Gln Asp Asn Pro Val Met Glu Gly Phe
   145                 150                 155                 160
   Thr Pro Ile Val Gly Ile Asp Val Trp Glu His Ala Tyr Tyr Leu Lys
                   165                 170                 175
   Tyr Gln Asn Arg Arg Ala Asp Tyr Leu Gln Ala Ile Trp Asn Val Leu
               180                 185                 190
   Asn Trp Asp Val Ala Glu Glu Phe Phe Lys Lys Ala
           195                 200